**Europäisches Patentamt**

**European Patent Office**

**Office européen des brevets**

(11) Veröffentlichungsnummer : **0 037 510**
**B1**

# EUROPÄISCHE PATENTSCHRIFT

(45) Veröffentlichungstag der Patentschrift :
**09.03.83**

(21) Anmeldenummer : **81102195.5**

(22) Anmeldetag : **24.03.81**

(51) Int. Cl.³ : **C 07 C143/60**

(54) Verfahren zur Herstellung von 1-Naphthylamin-4,6-disulfonsäure und 1-Naphthylamin-2,4,6-trisulfonsäure.

(30) Priorität : **05.04.80 DE 3013275**

(43) Veröffentlichungstag der Anmeldung :
**14.10.81 Patentblatt 81/41**

(45) Bekanntmachung des Hinweises auf die Patenterteilung : **09.03.83 Patentblatt 83/10**

(84) Benannte Vertragsstaaten :
**CH DE FR GB IT LI**

(56) Entgegenhaltungen :
**DE A 2 714 031**
**DE C 41 957**

**BEILSTEINS HANDBUCH DER ORGANISCHEN CHEMIE, vierte Auflage, Band XIV, Verlag von Julius Springer, 1931, Seiten 790, 802-803 Berlin, DE.**

(73) Patentinhaber : **BAYER AG**
**Zentralbereich Patente, Marken und Lizenzen**
**D-5090 Leverkusen 1, Bayerwerk (DE)**

(72) Erfinder : **Blank, Heinz Ulrich, Dr.**
**Am Geusfelde 35**
**D-5068 Odenthal (DE)**
Erfinder : **Behre, Horst, Dr.**
**Zur alten Linde 12**
**D-5068 Odenthal-Eikamp (DE)**
Erfinder : **Linden, Hans Werner, Dr.**
**Heymannstrasse 38**
**D-5090 Leverkusen (DE)**
Erfinder : **Mentzel, Werner, Dr.**
**Morgengraben 5**
**D-5000 Koeln 80 (DE)**

Verfahren zur Herstellung von 1-Naphthylamin-4.6-disulfonsäure und 1-Naphthylamin-2.4.6-trisulfonsäure.

Die Erfindung betrifft ein neues Verfahren zur Herstellung von 1-Naphthylamin-4.6-disulfonsäure und 1-Naphthylamin-2.4.6-trisulfonsäure.

1-Naphthylamin-4,6-disulfonsäure und 1-Naphthylamin-2,4,6-trisulfonsäure sind wichtige Zwischenprodukte für die Herstellung von Farbstoffen.

Es sind bereits verschiedene Verfahren zur Herstellung von 1-Naphthylamin-4.6-disulfonsäure bekannt. So ist z. B. in der FR-A-1 490 508 ein Verfahren zur Herstellung von 1-Naphthylamin-4.6-disulfonsäure beschrieben, gemäß dem man 1-Naphthylamin-6-sulfonsäure mit Mangandioxid und Natriumhydrogensulfit sulfoniert. Dieses Verfahren hat jedoch den Nachteil, daß teures Mangandioxid verwendet wird, eine schlechte Raum-Zeit-Ausbeute erzielt wird und große Mengen salzhaltiger Mutterlauge anfallen.

Es kommt daher für eine Herstellung von 1-Naphthylamin-4.6-disulfonsäure in technischem Maßstab nicht in Betracht.

Von technischem Interesse sind die Verfahren zur Herstellung von 1-Naphthylamin-4.6-disulfonsäure, bei denen die Sulfonierung von 1-Naphthylamin-6-sulfonsäure mit Oleum vorgenommen wird. Solche Verfahren sind z. B. in Beilstein H, XIV, Seite 790 ; Ullmann, Enzyklopädie der Technischen Chemie, Band 12, Seite 628 (1960) ; DE-P-Anmeldung C 4 021 (1891) referiert in Friedländer *33*, Seite 432 ; und der GB-PS 15 223 beschrieben.

Gemäß den Angaben in Beilstein soll sich 1-Naphthylamin-4.6-disulfonsäure durch Erhitzen von 1-Naphthylamin mit überschüssigem 25 %igen Oleum auf 120 °C oder durch mehrtägige Einwirkung von 25 %igem Oleum auf 1-Naphthylamin-4-sulfonsäure bei Temperatur unter 30 °C bilden. Gemäß Ullmann wird 1-Naphthylamin-4.6-disulfonsäure durch Eintragen von 1-Naphthylamin-4-sulfonsäure in 25 %iges Oleum und 25-stündiges Nachrühren bei 30 °C erhalten. Gemäß der in Friedländer referierten deutschen Patentanmeldung C 4 021 wird 1-Naphthylamin-4,6-disulfonsäure durch Behandeln von 1-Naphthylamin-6-sulfonsäure mit etwa 10 %igem Oleum bei 100-150 °C erhalten. Diese Verfahren haben jedoch den Nachteil, daß bei ihnen schwer trennbare Gemische aus beispielsweise 1-Naphthylamin-4.6- und -4,7-disulfonsäure entstehen.

Gemäß dem in der GB-A-15 223 beschriebenen Verfahren wird 1-Naphthylamin-6-sulfonsäure durch Eintragen in 25 %iges Oleum und nachfolgendes 3 bis 4-stündiges Wärmen der Sulfonierungsmischung auf 50 bis 60 °C sulfoniert. 1-Naphthylamin-4.6-disulfonsäure wird jedoch nicht isoliert ; das Sulfonierungsgemisch wird vielmehr unmittelbar weiter zu 1-Naphthylamin-2.4.6-trisulfonsäure sulfoniert. Die Nacharbeitung dieses Sulfonierungsverfahrens ergab, daß bei diesem beträchtliche Mengen an unerwünschter 1-Naphthylamin-3,6-disulfonsäure und 1-Naphthylamin-2.4.6-trisulfonsäure entstehen. Wegen der schlechten Ausbeuten an der gewünschten 1-Naphthylamin-4.6-disulfonsäure und der Schwierigkeiten bei der Isomerentrennung kommt daher diesem Verfahren für eine technische Produktion keine Bedeutung zu.

Es wurde nun gefunden, daß man zu einem auch in technischem Maßstab ausführbaren Verfahren zur Herstellung von 1-Naphthylamin-4.6-disulfonsäure gelangt, wenn man die Sulfonierung von 1-Naphthylamin-6-sulfonsäure unter bestimmten Bedingungen vornimmt. Die bestimmten Bedingungen bestehen darin, daß man a) das Sulfonierungsagenz Oleum zu in Schwefelsäure gelöster, bzw. suspendierter 1-Naphthylamin-6-sulfonsäure, bzw. gleichzeitig mit 1-Naphthylamin-6-sulfonsäure in vorgelegte Schwefelsäure gibt und b) ein solches Molverhältnis Schwefeltrioxid : 1-Naphthylamin-6-sulfonsäure anwendet, daß 1,2 bis 3 Mol Schwefeltrioxid auf 1 Mol 1-Naphthylamin-6-sulfonsäure entfallen. Durch die erfindungsgemäße Kombination dieser beiden speziellen Verfahrensmaßnahmen wird eine Steigerung der Ausbeuten an 1-Naphthylamin-4.6-disulfonsäure um etwa 20 %, verglicher mit der Ausbeute, die man nach dem günstigsten Verfahren gemäß Stand der Technik erhält, erreicht. Außerdem enthält die 1-Naphthylamin-4.6-disulfonsäure nur noch vernachlässigbare Spuren an unerwünschter 1-Naphthylamin-3,6-disulfonsäure.

Die Erfindung betrifft daher ein Verfahren zur Herstellung von 1-Naphthylamin-4.6-disulfonsäure durch Sulfonieren von 1-Naphthylamin-6-sulfonsäure mit Oleum, Aufarbeiten des Sulfonierungsgemisches durch Eintragen in Wasser und Isolieren der 1-Naphthylamin-4.6-disulfonsäure, das dadurch gekennzeichnet ist, daß man das Oleum bei einer Temperatur von 10 bis 70 °C, vorzugsweise 20 bis 40 °C, entweder gleichzeitig mit der 1-Naphthylamin-6-sulfonsäure zu vorgelegter Schwefelsäure gibt oder, vorzugsweise, zu in Schwefelsäure gelöster, bzw. suspendierter 1-Naphthylamin-6-sulfonsäure gibt und ein solches Molverhältnis Schwefeltrioxid : 1-Naphthylamin-6-sulfonsäure anwendet, daß 1,2 bis 3 Mol, vorzugsweise 1,5 bis 2,5 Mol, Schwefeltrioxid auf 1 Mol 1-Naphthylamin-6-sulfonsäure entfallen.

Das in dem erfindungsgemäßen Verfahren zu verwendende Oleum kann 20 bis 100 Gew.-% Schwefeltrioxid enthalten. Vorzugsweise wird ein Oleum verwendet, das 50 bis 80 Gewichtsprozent freies Schwefeltrioxid enthält.

Zum Lösen, bzw. Suspendieren der 1-Naphthylamin-6-sulfonsäure wird vorzugsweise 80 bis 100 %ige, insbesondere 96 bis 100 %ige Schwefelsäure verwendet. Die Menge an zum Lösen, bzw. Suspendieren verwendeter Schwefelsäure wird vorteilhaft so bemessen, daß auf 1 Mol 1-Naphthylamin-6-sulfonsäure 6 bis 8 Mol Schwefelsäure entfallen.

0 037 510

Wird zum Lösen, bzw. Suspendieren wasserhaltige Schwefelsäure verwendet, oder keine trockene sondern feuchte 1-Naphthylamin-6-sulfonsäure eingesetzt, so ist über die Menge von 1,2 bis 3 Mol Schwefeltrioxid je Mol 1-Naphthylamin-6-sulfonsäure hinaus so viel Schwefeltrioxid (in Form von Oleum) zuzusetzen, wie zum Binden des in den Reagenzien enthaltenden Wassers als Schwefelsäure erforderlich ist.

Nach erfolgter Zugabe des Oleums läßt man das Reaktionsgemisch bei Temperaturen von 20 bis 40 °C, vorzugsweise 25 bis 35 °C unter Rühren ausreagieren. Die Ausreagierzeit liegt zwischen 3 bis 30 Stunden, vorzugsweise 8 bis 24 Stunden.

Die 1-Naphthylamin-4.6-disulfonsäure wird aus dem fertigen Sulfonierungsgemisch in an sich bekannter Weise durch Eintragen des Gemisches in Wasser und Abfiltrieren der auskristallierten Säure, bzw. nach Aussalzen, z. B. mit Kochsalz, des sauren Mononatriumsalzes, erhalten.

Für den Fall der Weiterverarbeitung der 1-Naphthylamin-4.6-disulfonsäure zu 1-Naphthylamin-2.4.6-trisulfonsäure ist eine Isolierung der 1-Naphthylamin-4.6-disulfonsäure nicht erforderlich. Vielmehr wird das fertige Sulfonierungsgemisch in einer zweiten Sulfonierungsstufe bei Temperaturen von 70 bis 120 °C, vorzugsweise 80 bis 100 °C, mit einer solchen Menge Oleum versetzt, daß auf 1 Mol ursprünglich eingesetzte 1-Naphthylamin-6-sulfonsäure 1 bis 2,5 Mol, vorzugsweise 1,5 bis 2 Mol, Schwefeltrioxid entfallen. Nach beendeter Oleumzugabe läßt man das Sulfonierungsgemisch 2 bis 8 Stunden bei 70 bis 120 °C, vorzugsweise 80 bis 100 °C, ausreagieren. Man isoliert die entstandene 1-Naphthylamin-2.4.6-trisulfonsäure in an sich bekannter Weise durch Eintragen des Sulfonierungsgemisches in Wasser. Die Sulfonsäure wird als freie Säure, bzw. durch Aussalzen, z. B. mit Kochsalz, als saures Dinatriumsalz erhalten.

Infolge ihres wesentlich höheren Gehaltes an 1-Naphthylamin-4.6-disulfonsäure und geringem Gehalt an 1-Naphthylamin-3.6-disulfonsäure liefert das bei der erfindungsgemäßen Herstellung von 1-Naphthylamin-4.6-disulfonsäure anfallende Sulfonierungsgemisch 1-Naphthylamin-2.4.6-trisulfonsäure in wesentlich höheren Ausbeuten und höherer Reinheit als die gemäß Stand der Technik erhältlichen, 1-Naphthylamin-4.6-disulfonsäure enthaltenden Sulfonierungsgemische.

Die Erfindung betrifft daher fernerhin ein Verfahren zur Herstellung von 1-Naphthylamin-2,4,6-trisulfonsäure.

Das Verfahren ist dadurch gekennzeichnet, daß man das bei der Herstellung von 1-Naphthylamin-4.6-disulfonsäure anfallende Sulfonierungsgemisch in einer zweiten Sulfonierungsstufe bei Temperaturen von 70 bis 120 °C, vorzugsweise 80 bis 100 °C, mit Oleum umsetzt, wobei man die Oleummenge so bemißt, daß 1 bis 2,5 Mol, vorzugsweise 1,5 bis 2 Mol Schwefeltrioxid auf 1 Mol ursprünglich eingesetzte 1-Naphthylamin-6-sulfonsäure entfallen.

Das zu verwendende Oleum kann 20 bis 100 Gew.-% Schwefeltrioxid enthalten. Vorzugsweise wird ein Oleum verwendet, das 50 bis 80 Gewichtsprozent freies Schwefeltrioxid enthält.

Beispiel 1

In einem mit Rührer, Tropftrichter, Innenthermometer und Trockenrohr versehenen Reaktionsgefäß werden 700 g 100 %ige Schwefelsäure vorgelegt. In diese werden unter Rühren innerhalb von 30 Minuten 226,8 g (1 Mol) 1-Naphthylamin-6-sulfonsäure (98 %ig) bei höchstens 40 °C eingetragen. Die Mischung wird solange gerührt, bis eine Lösung entstanden ist. Dann werden innerhalb von 30 Minuten bei 30 °C 246 g Oleum (65 %ig) (= 2 Mol $SO_3$) zugetropft. Die Sulfonierungsmischung wird 16 Stunden bei 30 °C nachgerührt.

Dann wird das Sulfonierungsgemisch unter Rühren mit einer solchen Geschwindigkeit in 1 434 g Wasser eingetragen, daß die Temperatur der entstehenden wässrigen Mischung auf 90 bis 95 °C steigt. Nach wenigen Minuten fällt die 1-Naphthylamin-4.6-disulfonsäure aus. Man läßt unter Rühren auf 20 °C abkühlen (Abkühlzeit : 5 bis 6 Stunden) und saugt ab. Der Filterkuchen wird gut abgepresst.

Die Ausbeute an 1-Naphthylamin-4.6-disulfonsäure beträgt 321 g (= 80,6 % der Theorie bezogen auf eingesetzte 1-Naphthylamin-6-sulfonsäure).

Die Hochdruckflüssigkeitschromatographie des abfiltrierten Produktes ergab folgende Zusammensetzung :

1-Naphthylamin-4.6-disulfonsäure : 76,1 Gew.-%
1-Naphthylamin-3.6-disulfonsäure : 0,1 Gew.-%
1-Naphthylamin-2.4.6-trisulfonsäure : Spur
Rest bis 100 % : Wasser/Schwefelsäure

Beispiele 2 bis 5

Es wird wie in Beispiel 1 beschrieben, gearbeitet, nur werden andere Molverhältnisse Schwefeltrioxid : 1-Naphthylamin-6-sulfonsäure, andere Reaktionstemperaturen und Nachrührzeiten angewendet.

In der nachstehenden Tabelle sind die bei diesen Herstellungsverfahren erhaltenen Ergebnisse zusammengestellt. Die Zusammensetzung der isolierten 1-Naphthylamin-4.6-disulfonsäure wurde mittels Hochdruck-Flüssigkeitschromatographie bestimmt.

3

TABELLE

0 037 510

| Bei-spiel Nr. | Sulfonierungsbedingu   n | | | | Isolierter Feststoff Zusammensetzung (Gew.-%)* | | | | Ausbeute an 1-NH$_2$-4.6 (Mol-%) |
| | Molver-hältnis SO$_3$:1-NH$_2$-6-S | Reaktions-temperatur (°C) | Nach-rühr-zeit (h) | Aus-wage (g) | 1-NH$_2$-6 | 1-NH$_2$-3.6 | 1-NH$_2$-4.6 | 1-NH$_2$-2.4.6 | |
|---|---|---|---|---|---|---|---|---|---|
| 2 | 2,5:1 | 30 | 9 | 351 | Spur | 2,8 | 68,5 | Spur | 79,2 |
| 3 | 2 :1 | 40 | 8 | 308 | Spur | 0,6 | 76,7 | 0,2 | 77,9 |
| 4 | 2 :1 | 50 | 3 | 301 | Spur | 0,5 | 78,0 | 0,7 | 77,5 |
| 5 | 1,75:1 | 30 | 24 | 367 | Spur | 1,8 | 65,9 | Spur | 79,7 |

* Bestimmt mittels Hochdruck-Flüssigkeitschromatographie ; bei den an 100 % fehlenden Gew.-% handelt es sich um $H_2O$ und $H_2SO_4$.

**0 037 510**

Beispiel 6 (Sulfonierung gemäß GB-PS 15 223)

In der im Beispiel 1 verwendeten Sulfonierungsapparatur werden 780 g (2,44 Mol SO$_3$) Oleum (25 %ig) vorgelegt. In dieses werden bei 20 °C innerhalb von 30 Minuten 225 g (1 Mol) 1-Naphthylamin-6-sulfonsäure (99 %ig) eingetragen. Anschließend wird das Sulfonierungsgemisch 4 Stunden bei 50 °C nachgerührt.

Die durch Hochdruck-Flüssigkeitschromatographie des fertigen Sulfonierungsproduktes bestimmte Ausbeute an 1-Naphthylamin-4.6-disulfonsäure beträgt 62 % der Theorie, bezogen auf eingesetzte 1-Naphthylamin-6-sulfonsäure.

Gemäß Hochdruck-Flüssigkeitschromatographie weist das Sulfonierungsgemisch folgende Zusammensetzung auf :

| | |
|---|---|
| 1-Naphthylamin-4.6-disulfonsäure : | 18,7 Gew.-% (62,0 Mol-%) |
| 1-Naphthylamin-3.6-disulfonsäure : | 7,49 Gew.-% (24,8 Mol-%) |
| 1-Naphthylamin-2.4.6-trisulfonsäure : | 3,44 Gew.-% (9,0 Mol-%) |

Beispiel 7

In der in Beispiel 1 verwendeten Sulfonierungsapparatur werden 700 g 100 %ige Schwefelsäure vorgelegt. In diese werden 225 g (1 Mol) 1-Naphthylamin-6-sulfonsäure (99 %ig) bei einer Temperatur von höchstens 40 °C eingetragen. In diese Mischung werden innerhalb von 30 Minuten bei 30 °C 246 g 65 %iges Oleum (= 2 Mol SO$_3$) getropft. Die Sulfonierungsmischung wird anschließend 16 Stunden bei 30 °C gerührt.

Dann werden nochmals 246 g 65 %iges Oleum (= 2 Mol SO$_3$) zugegeben. Das Sulfonierungsgemisch wird unter Rühren 6 Stunden auf 90 °C erwärmt, dann auf 20 bis 30 °C abgekühlt und dann mit einer solchen Geschwindigkeit in 2 600 g Wasser eingetragen, daß die Temperatur der entstehenden wässrigen Mischung auf 95 °C ansteigt. Man versetzt die wässrige Mischung mit 250 g Kochsalz und kühlt im Verlauf von 5 bis 6 Stunden auf 20 °C, saugt den Niederschlag ab und wäscht ihn mit gesättigter Kochsalzlösung, bis diese annähernd farblos abläuft. Der Feststoff wird im Vakuum-Trockenschrank bei 90 °C getrocknet.

Die Ausbeute an 1-Naphthylamin-2.4.6-trisulfonsäuredinatriumsalz beträgt 553 g = 87,7 % der Theorie bezogen auf 1-Naphthylamin-6-sulfonsäure.

Das abfiltrierte Produkt weist gemäß Hochdruck-Flüssigkeitschromatographie folgende Zusammensetzung auf :

1-Naphthylamin-2.4.6-trisulfonsäure : 60,7 Gew.-%
1-Naphthylamin-3.6-disulfonsäure : 1,15 Gew.-%
1-Naphthylamin-4.6-disulfonsäure : 0,2 Gew.-%
Rest bis 100 % : NaCl/H$_2$O

Beispiel 8 (Sulfonierung gemäß GB-PS 15 223)

In der in Beispiel 1 verwendeten Sulfonierungsapparatur werden 780 g 25 %iges Oleum (= 2,44 Mol SO$_3$) vorgelegt. In dieses werden unter Kühlen bei 20 °C innerhalb von 30 Minuten 225 g (1 Mol) 1-Naphthylamin-6-sulfonsäure (99 %ig) eingetragen. Anschließend wird das Sulfonierungsgemisch 4 Stunden auf 50 °C erwärmt. Dann werden 240 g 65 %iges Oleum (= 1,95 Mol SO$_3$) rasch zugetropft. Die Mischung wird 4,5 Stunden auf 90 °C erwärmt. Dann wird das Sulfonierungsgemisch auf 20 bis 30 °C abgekühlt und so rasch in 2 600 g Wasser eingetragen, daß die Temperatur der wässrigen Mischung auf etwa 95 °C ansteigt. Man setzt der wässrigen Mischung 250 g Kochsalz zu und lässt sie im Verlauf von etwa 5 bis 6 Stunden auf 20 °C abkühlen. Der ausgefallene Feststoff wird abgesaugt und mit gesättigter Kochsalzlösung gewaschen, bis die ablaufende Kochsalzlösung fast farblos ist. Der Feststoff wird im Vakuum-Trockenschrank bei 90 °C getrocknet.

Die Ausbeute an 1-Naphthylamin-2.4.6-trisulfonsäure-dinatriumsalz beträgt 506 g (= 57,5 % der Theorie bezogen auf 1-Naphthylamin-6-sulfonsäure).

Gemäß Hochdruck-Flüssigkeitschromatographie weist das isolierte Produkt folgende Zusammensetzung auf :

1-Naphthylamin-2.4.6-trisulfonsäure : 43,5 Gew.-%
1-Naphthylamin-3.6-disulfonsäure : 7,5 Gew.-%
1-Naphthylamin-4.6-disulfonsäure : 0,15 Gew.-%
Rest bis 100 % : H$_2$O/NaCl

5

**Ansprüche**

1. Verfahren zur Herstellung von 1-Naphthylamin-4.6-disulfonsäure durch Sulfonieren von 1-Naphthylamin-6-sulfonsäure mit Oleum, Aufarbeiten des Sulfonierungsgemisches durch Eintragen in Wasser und Isolieren der 1-Naphthylamin-4.6-disulfonsäure, dadurch gekennzeichnet, daß man das Oleum bei einer Temperatur von 10 bis 70 °C entweder gleichzeitig mit der 1-Naphthylamin-6-sulfonsäure zu vorgelegter Schwefelsäure gibt oder zu in Schwefelsäure gelöster, bzw. suspendierter 1-Naphthylamin-6-sulfonsäure gibt und ein solches Molverhältnis Schwefeltrioxid zu 1-Naphthylamin-6-sulfonsäure anwendet, daß 1,2 bis 3-Mol Schwefeltrioxid auf 1 Mol 1-Naphthylamin-6-sulfonsäure entfallen.

2. Verfahren gemäß Anspruch 1, dadurch gekennzeichnet, daß man ein solches Molverhältnis Schwefeltrioxid zu 1-Naphthylamin-6-sulfonsäure anwendet, daß 1,5 bis 2,5 Mol Schwefeltrioxid auf 1 Mol 1-Naphthylamin-6-sulfonsäure entfallen.

3. Verfahren nach Anspruch 1, und 2, dadurch gekennzeichnet, daß man zum Vorlegen bzw. Lösen bzw. Suspendieren 80 bis 100 %ige Schwefelsäure verwendet.

4. Verfahren nach Anspruch 1 bis 3, dadurch gekennzeichnet, daß man die 80 bis 100 %ige Schwefelsäure in einer solchen Menge anwendet, daß auf 1 Mol 1-Naphthylamin-6-sulfonsäure 6 bis 8 Mol Schwefelsäure entfallen.

5. Verfahren gemäß Anspruch 1 bis 4, dadurch gekennzeichnet, daß man das Oleum bei Temperaturen von 20 bis 40 °C zugibt.

6. Verfahren zur Herstellung von 1-Naphthylamin-2.4.6-trisulfonsäure, dadurch gekennzeichnet, daß man das bei der Herstellung von 1-Naphthylamin-4.6-disulfonsäure gemäß Anspruch 1 bis 4 anfallende Sulfonierungsgemisch in einer zweiten Sulfonierungsstufe bei Temperaturen von 70 bis 120 °C mit Oleum umsetzt, wobei man die Oleum-Menge so bemißt, daß 1 bis 2,5 Mol Schwefeltrioxid auf 1 Mol ursprünglich eingesetzte 1-Naphthylamin-6-sulfonsäure entfallen.

**Claims**

1. Process for the preparation of 1-naphthylamine-4,6-disulphonic acid by sulphonating 1-naphthylamine-6-sulphonic acid with oleum, working up the sulphonation mixture by introducing it into water, and isolating the 1-naphthylamine-4,6-disulphonic acid, characterised in that, at a temperature of 10 to 70 °C, either the oleum is added to initially introduced sulphuric acid simultaneously with the 1-naphthylamine-6-sulphonic acid or the oleum is added to 1-naphthylamine-6-sulphonic acid, which is dissolved or suspended in sulphuric acid, and a molar ratio of sulphur trioxide to 1-naphthylamine-6-sulphonic acid such that 1.2 to 3 mols of sulphur trioxide are present per mol of 1-naphthylamine-6-sulphonic acid, is used.

2. Process according to Claim 1, characterised in that a molar ratio of sulphur trioxide to 1-naphthylamine-6-sulphonic acid such that 1.5 to 2.5 mols of sulphur trioxide are present per mol of 1-naphthylamine-6-sulphonic acid is used.

3. Process according to Claims 1 and 2, characterised in that 80 to 100 % strength sulphuric acid is used as the initial component or for obtaining the solution or suspension.

4. Process according to Claims 1 to 3, characterised in that the 80 to 100 % strength sulphuric acid is used in an amount such that 6 to 8 mols of sulphuric acid are present per mol of 1-naphthylamine-6-sulphonic acid.

5. Process according to Claims 1 to 4, characterised in that the oleum is added at temperatures of 20 to 40 °C.

6. Process for the preparation of 1-naphthylamine-2,4,6-trisulphonic acid, characterised in that the sulphonation mixture obtained in the preparation of 1-naphthylamine-4,6-disulphonic acid according to Claims 1 to 4 is reacted, in a second sulphonation stage, with oleum at temperatures of 70 to 120 °C, the amount of oleum being such that 1 to 2.5 mols of sulphur trioxide are present per mol of 1-naphthylamine-6-sulphonic acid originally employed.

**Revendications**

1. Procédé de production d'acide 1-naphtylamine-4.6-disulfonique par sulfonation d'acide 1-naphtylamine-6-sulfonique avec l'oléum, traitement du mélange de sulfonation par addition d'eau et isolement de l'acide 1-naphtylamine-4.6-disulfonique, caractérisé en ce qu'on ajoute l'oléum, à une température de 10 à 70 °C, en même temps que l'acide 1-naphtylamine-6-sulfonique à de l'acide sulfurique introduit au préalable ou bien à de l'acide 1-naphtylamine-6-sulfonique dissous ou en suspension dans l'acide sulfurique et on utilise un rapport molaire de l'anhydride sulfurique à l'acide 1-naphtylamine-6-sulfonique choisi de manière qu'il y ait 1,2 à 3 moles d'anhydride sulfurique· par mole d'acide 1-naphtylamine-6-sulfonique.

2. Procédé suivant la revendication 1, caractérisé en ce qu'on utilise un rapport molaire de l'anhydride sulfurique à l'acide 1-naphtylamine-6-sulfonique choisi de manière qu'il y ait 1,5 à 2,5 moles d'anhydride sulfurique par mole d'acide 1-naphtylamine-6-sulfonique.

3. Procédé suivant les revendications 1 et 2, caractérisé en ce qu'on utilise pour l'introduction préalable ou la dissolution ou la mise en suspension de l'acide sulfurique à 80-100 %.

4. Procédé suivant les revendications 1 à 3, caractérisé en ce qu'on utilise de l'acide sulfurique à 80-100 % en une quantité choisie de manière qu'il y ait par mole d'acide 1-naphtylamine-6-sulfonique 6 à 8 moles d'acide sulfurique.

5. Procédé suivant les revendications 1 à 4, caractérisé en ce qu'on ajoute l'oléum à des températures de 20 à 40 °C.

6. Procédé de production d'acide 1-naphtylamine-2.4.6-trisulfonique, caractérisé en ce qu'on fait réagir le mélange de sulfonation obtenu lors de la production d'acide 1-naphtylamine-4.6-disulfonique conformément aux revendications 1 à 4 dans une seconde étape de sulfonation à des températures de 70 à 120 °C avec l'oléum dont on mesure la quantité de manière qu'il y ait 1 à 2,5 moles d'anhydride sulfurique par mole d'acide 1-naphtylamine-6-sulfonique initialement utilisé.